# EUROPEAN PATENT SPECIFICATION

(11) **EP 2 124 828 B1**
(45) Date of publication and mention of the grant of the patent: **23.04.2014**
(21) Application number: 08718891.8
(22) Date of filing: 27.03.2008
(51) Int. Cl.: A61F 2/36

(54) **AN ASSEMBLY FOR USE IN A HIP JOINT REPLACEMENT PROCEDURE**
INSTRUMENT ZUR VERWENDUNG IN EINEM HÜFTGELENKERSETZUNGSVERFAHREN
ENSEMBLE CONÇU POUR LE REMPLACEMENT D'UNE ARTICULATION DE LA HANCHE

(30) Priority: 28.03.2007 GB 0705901
(43) Date of publication of application: 02.12.2009
(73) Proprietor: DePuy International Limited, Leeds LS11 8DT (GB)
(72) Inventor: BROOKS, James, Beeston, Leeds LS11 8DT (GB); THOMPSON, Jonathan, Beeston, Leeds LS11 8DT (GB)
(74) Representative: Belcher, Simon James
(86) International application number: PCT/GB2008/001057
(87) International publication number: WO 2008/117057

(56) References cited:
- EP-A- 1 285 639
- WO-A-01/22905
- WO-A-97/25943
- WO-A-2004/030581
- FR-A- 2 773 469
- US-A1- 2004 143 335
- US-A1- 2005 197 708
- US-A1- 2007 050 040

## Description

This invention relates to an assembly for use in a hip joint replacement procedure.

Hip joint prostheses generally comprise a femoral component which has a medially directed neck with a polished head at its medial end. The head articulates in a cup component which is implanted in the patient's acetabulum.

The success of the implantation procedure depends in part on the appropriate alignment of the head and cup components. Inappropriate alignment can give rise to post-operative problems, including limited range of motion, wear of the bearing surfaces which is even or excessive, and damage to soft tissue which is associated with the joint.

The distance by which the bearing surface of the femoral head is offset relative to the femoral axis determines the effective leg length and soft tissue tension across the joint resulting from the procedure. That distance can be selected to suit the requirements of a particular patient by selection of an appropriate prosthesis, or by selection of an appropriate modular part of a component which comprises several modular parts (for example modular head and stem parts).

The present invention provides a femoral component in which the end of the stem component is located relatively close to the femoral head bearing surface, resulting in a short effective femoral neck length.

Accordingly, in one aspect, the invention provides an assembly for use in a hip joint replacement procedure, which comprises:
a. a stem part for location in the intramedullary cavity of a femur, having a tapered spigot at one end,
b. a head part having a bearing surface defined by a part of a sphere which subtends an angle at the center of the sphere of at least about 108°, and a tapered bore formed in it in which the spigot is a snug fit so that the tapered surfaces of the spigot and the socket are in surface to surface contact,
in which, when the spigot is received in the bore in the head part, the distance from (i) the point on the tapered surface of the spigot at or towards its free end at which the tapered surface thereof no longer contacts the tapered surface of the bore, to (ii) the centre of the sphere which is defined by the bearing surface measured from the said point on the spigot parallel to the axis of the spigot towards the intramedullary cavity, is at least about 5 mm, with the said point on the tapered surface of the spigot being located between the centre of the sphere and the bearing surface of the head part.

In another aspect, the invention provides an assembly for use in a hip joint replacement procedure, which comprises:
a. a stem part for location in the intramedullary cavity of a femur, having a tapered spigot at one end,
b. a head part having a bearing surface defined by a part of a sphere which subtends an angle at the centre of the sphere of at least about 180°, and a tapered bore formed in it in which the spigot can be received,
in which, when the spigot is received in the bore in the head part, the ratio of (i) the distance from the point on the spigot at or towards its free end where the tapered surface thereof contacts the tapered surface of the bore to the centre of the sphere which is defined by the bearing surface, measured from the said point on the spigot parallel to the axis of the spigot towards the intramedullary cavity, to (ii) the radius of the sphere which is defined by the bearing surface, is at least about 0.3.

Preferably, the distance from (i) the point on the tapered surface of the spigot at or towards its free end at which the tapered surface thereof no longer contacts the tapered surface of the bore, to (ii) the centre of the sphere which is defined by the bearing surface measured from the said point on the spigot parallel to the axis of the spigot towards the intra-medullary cavity, is at least about 8 mm, more preferably at least about 10 mm, for example at least about 12 mm.

Preferably, the ratio of (i) the distance from the point on the spigot at or towards its free end where the tapered surface thereof contacts the tapered surface of the bore to the centre of the sphere which is defined by the bearing surface, measured from the said point on the spigot parallel to the axis of the spigot towards the intramedullary cavity, to (ii) the radius of the sphere which is defined by the bearing surface, is at least about 0.5, more preferably at least about 0.6.

The assembly of the invention has the advantage that it enables different femoral component configurations to be achieved. This can be facilitated for example when the femoral head has a relatively large diameter, for example a diameter which is at least about 7 mm, preferably at least about 10 mm, more preferably at least about 15 mm, especially in some embodiments at least about 20 mm such as at least about 30 mm or at least about 35 mm. Positioning the end of the spigot provided on the neck of the femoral component further from the centre of the sphere which is defined by the femoral bearing surface is facilitated with such larger size femoral heads. For example, it means that the end of the spigot can be positioned closer to the bearing surface of the head part without compromising the thickness of the material of the head part and therefore the ability of the head part to carry a load after implantation.

The tapered surface of the spigot will often extend to the end of the spigot so that the plane on which the spigot is truncated intersects the said tapered surface. The measurement between the centre of the sphere and the point on the tapered surface of the spigot at or towards its free end at which the tapered surface thereof no longer contacts the tapered surface of the bore will then generally be made to the end of the tapered surface of the spigot. However, the point at which the tapered surface no longer contacts the tapered surface of the bore might be spaced from the end of the spigot, for example, when the bore is flared outwardly beyond the point at which the tapered surfaces contact one another, or where the spigot is tapered inwardly beyond that point.

Preferably, the thickness of the material of the head part is not less than about 3 mm, more preferably not less than about 3 mm, for example not less than about 4 mm, over a circular area around the pole which subtends an angle of 20° at the centre of the sphere which is defined by the bearing surface.

It can be preferred to use a connector part, positioned between the stem part and the head part, to connect the head part to the stem part. For example, the assembly of the invention can comprise:
a. a head part having the shape of a truncated sphere with a convex bearing surface which can engage and articulate with the socket component and an opposite reverse face, the head part having a blind bore with a circular cross section within it located eccentrically relative to the axis of the head part, in which the bore extends into the head part from an opening on the reverse face and is inwardly tapered continuously from the opening towards the blind end thereof,
b. a stem part having a distal end and a head end, which can be fitted distal end first into a bone cavity, and
c. a connector part with a circular cross-section which is tapered inwardly along the axis defined by its external surface so that it can be received snugly in the tapered bore in the head part,
in which the connector part is provided with a first connection feature comprising one of an inwardly tapered bore and a spigot, with a circular cross section located eccentrically relative to the axis of the connector part, and the stem part has a second connection feature comprising the other of an inwardly tapered bore and a spigot, in which the spigot is a snug fit in the bore to connect the stem part to the connector part,
and in which the ratio of the length of the contacting surfaces of the bore in the head part and the connector part when assembled, measured along the axis of the bore in the head part, to the diameter of the bore in the head part at the widest point at which it contacts the external surface of the connector part, is at least about 0.9, preferably at least about 1.0, for example at least about 1.2, preferably at least about 1.5, for example at least about 2.0.

When the assembly includes a connector part as mentioned above, the connector part, when mounted on the stem part, can provide the spigot which is received in the bore in the head part.

The use of a connector part in the assembly of the present invention has the advantage that it can enable variation of effective leg length and the extent of retroversion and anteversion in the assembled prosthesis. This has been found to be possible using a connector part, provided that the ratio of the length of the contacting surfaces of the bore in the head part and the connector part when assembled, measured along the axis of the bore in the head part, to the diameter of the bore in the head part at the widest point at which it contacts the external surface of the connector part, is at least about 0.9, preferably at least about 1.0, for example at least about 1.2, preferably at least about 1.5, for example at least about 2.0. This feature has been found to enable the component to withstand the loads which are imposed on a hip joint prosthesis after implantation, especially having regard to the fact that the greatest loads are applied in a direction which is not aligned with the axis of the neck.

Preferably, the ratio of the length of the contacting surfaces of the first and second connection features, measured along the axis of the bore, to the diameter of the bore at the widest point at which it contacts the external surface of the spigot, is at least about 0.9, preferably at least about 1.0, for example at least about 1.2, preferably at least about 1.5, for example at least about 2.0.

The reverse face of the head part will often be approximately planar. In particular, it can be preferred that the opening to the bore in the head part falls in a plane. Preferably, the plane defined by the opening to the bore in the head part is approximately perpendicular to the axis of the bore. Preferably, the plane defined by the opening to the bore in the head part is approximately perpendicular to the axis of the head part.

It will generally be preferred that the ratio of the depth of the head part which provides the bearing surface measured along the axis of the head part, to its diameter measured perpendicular to the axis of the head part at its widest point, is at least about 0.5, for example at least about 0.6, or at least about 0.7, or at least about 0.8. This can provide adequate contact area between the articulating bearing surfaces of the prosthesis components across the range of motion of the joint.

Generally, the bearing surface of the head part is part-spherical with an approximately constant radius. Preferably, the radius of the sphere which defines the bearing surface is at least about 7 mm, more preferably at least about 9 mm, for example at least about 11 mm. Preferably, the radius is not more than about 30 mm, more preferably not more than about 20 mm, for example not more than about 15 mm or about 12 mm.

Preferably, the angle of arc through which the bearing surface of the head part extends (when the head part is viewed in cross-section on the plane which contains its axis) is more preferably at least about 190°, for example at least about 200°.

Preferably, the axis of the head part defined by its bearing surface and the axis of the bore in the head part are approximately parallel. The axis of the head part defined by its bearing surface and the axis of the bore in the head part can be approximately coincident. The distance between the axis of the head part defined by its bearing surface and the axis of the bore in the head part can be greater than zero, for example at least about 1 mm, preferably at least about 2 mm, more preferably at least about 5 mm. The distance between the axis of the head part defined by its bearing surface and the axis of the bore in the head part can be not more than about 10 mm, for example not more than about 8 mm, or not more than about 6 mm.

Preferably, the axis of the connector part defined by its external surface and the axis of the first connection feature are approximately parallel. The distance between the axis of the connector part defined by its external surface and the axis of the first connection feature can be at least about 1 mm, for example at least about 2 mm. The distance between the axis of the connector part defined by its external surface and the axis of the first connection feature can be not more than about 6 mm, for example not more than about 4 mm. Preferably, the diameter of the bore in the head part at the widest point at which it is contacted by the external surface of the connector part when assembled is not more than about 35 mm, more preferably not more than about 30 mm. Preferably, the diameter of the bore in the head part at the widest point at which it is contacted by the external surface of the connector part when assembled is at least about 10 mm, more preferably at least about 15 mm, for example at least about 20 mm.

It can be preferred for the connector part to have at least two inwardly tapered bores formed within it, each having a circular cross section, and each capable of receiving a spigot on the stem part in a snug fit, in which the axes of the bores are approximately and the distance from the axis of the connector part defined by its external surface to the axis of one of the bores is different from the distance from the axis of the connector part defined by its external surface to the axis of another of the bores. Selection of the bore in the connector part in which the spigot is inserted can be used to select the configuration of the assembled femoral component to suit the requirements of a patient.

It will generally be preferred for the stem part or the connector part when present or both to be formed from a metal. Suitable metals for use in the manufacture of one or each these parts include certain stainless steels, titanium and its alloys, and alloys which include cobalt and molybdenum.

It will generally be preferred for the head part of the component to be formed from one or more of a metal and a ceramic material. Suitable metals for use in the manufacture of one or each these parts include certain stainless steels, titanium and its alloys, and alloys which include cobalt and molybdenum. Suitable ceramic materials include certain oxides, nitrides and carbides of elements such as aluminium, zirconium, titanium and so on.

Embodiments of the invention will now be described by way of example with reference to the accompanying drawings, in which:
Figure 1 is a view of a prepared acetabulum, in which an acetabular cup component has been implanted.
Figure 2 is a view along the anterior posterior axis of the head of a femur, in which a stem part of a femoral component of a hip joint prosthesis has been implanted.
Figure 3 is a view from below of a head part of a femoral component.
Figure 4 is an isometric view from below of a connector in place which can be used to connect the head part shown in Figure 3 to the stem part shown in Figure 2.
Figure 5 is a view of the head of a femur to illustrate the offset of the bearing surface of the femoral head relative to the femoral neck.
Figure 6 is a view from below of a trial instrument which can be used to select the appropriate offset in an assembled femoral component.
Figure 7 is a side view of the trigger part of the trial instrument which is shown in Figure 6.

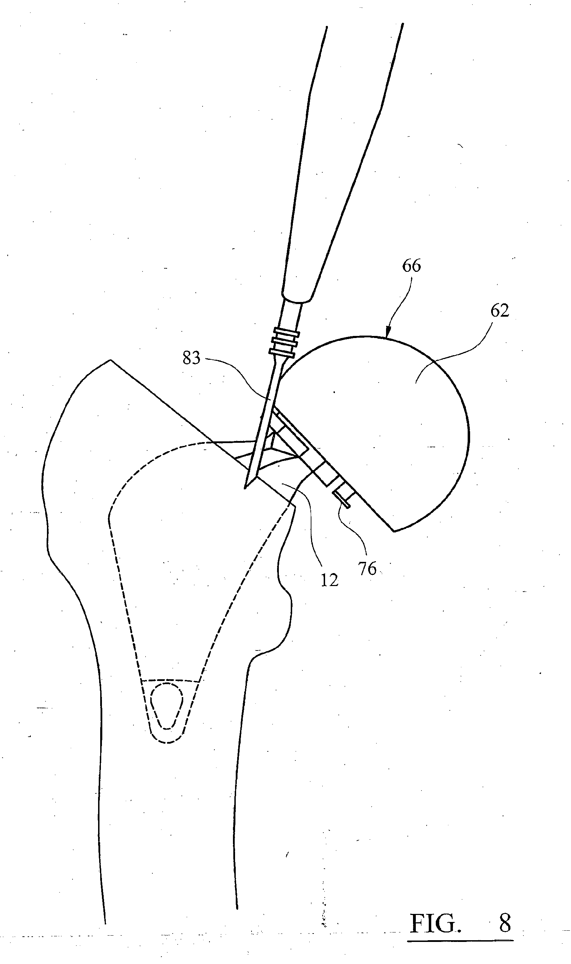

Figure 8 is a side view of the head of the femur, with the trial instrument shown in Figure 6 mounted on the stem part.
Figure 9 is a side view of a tool which can be used to assemble the head part and the connector, shown in Figures 3 and 4 respectively.
Figure 10 is a view from one side of an assembled femoral component of a hip joint prosthesis according to the present invention.

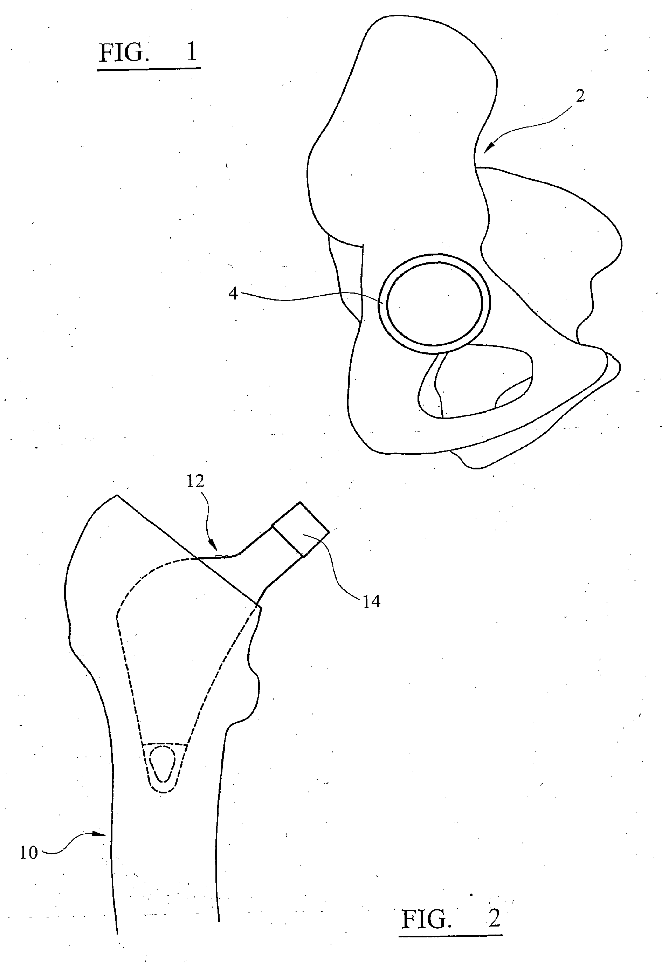

Referring to the drawings, Figure 1 shows a pelvis 2 which is been reamed to receive the acetabular cup component 4 of a hip joint prosthesis. The acetabular cup component has been implanted using conventional techniques.

Figure 2 shows the head portion of a femur 10 which has been resected at the base of the femoral neck. The intramedullary cavity has been prepared using conventional techniques (by reaming or broaching or a combination of the two) to receive the stem part 12 of the femoral component of a hip joint prosthesis. The stem part can be fastened in the femur by means of a bone cement material, as is known. The stem part can be fastened in the femur without the use of a bone cement material, as is known.

The stem part has a tapered spigot 14 at its exposed end on which the head part of the femoral component can be fitted. The dimensions of the spigot on the stem part are in line with existing stem parts of femoral components of hip joint prostheses.

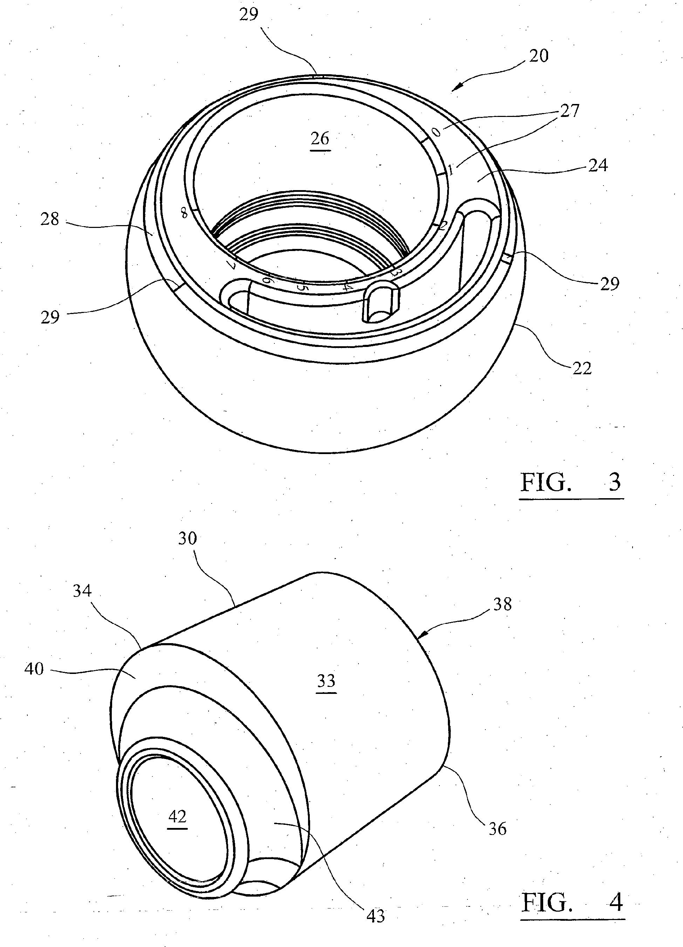

Figure 3 shows the head part 20 of a femoral component of a hip joint prosthesis according to the present invention. The head part has a spherical bearing surface 22 and an opposite reverse face 24. The spherical bearing surface extends through an angle of arc of about 200°. The radius of the bearing surface is 18 mm. The distance from the reverse face of the head part to the point where the polar axis intersects the bearing surface is from 28.25 to 41.8 mm.

A tapered bore 26 is formed in the reverse face 24. The bore has a circular cross-section. At the reverse face, the diameter of the bore is from 24.2 to 28.6 mm. The depth of the bore, measured from the reverse face of the head part to the blind end of the bore; is from 9.0 to 11.5 mm. The angle between the wall of the bore and its axis (which is half of the angle defined by the diametrically opposite walls of the bore) is 5°.

The bore 26 is offset relative to the polar axis (which is the axis extending through the centre of the sphere defined by the bearing surface, perpendicular to the reverse face). The distance between the axis of the bore and the polar axis is from 2 to 4 mm.

The head part has a series of markings 27 on its reverse face. These relate to the distance through which the head part is offset relative to the axis of the stem part when the femoral component is assembled, as discussed below.

The head component has a chamfer surface 28 extending around its periphery where the chamfer and reverse faces come together. The chamfer surface is planar when the component is viewed in cross-section. The angle between the chamfer surface and the polar axis is about 50°. The chamfer surface has three markings 29 at spaced apart points. The markings are distinguishable from one another.

Figure 4 shows a connector 30 which can be used to connect the head part to the stem part 12 of the femoral component. The connector is circular when viewed from above and is tapered inwardly along the axis defined by its external surface 32. The diameter of the connector at its widest 34 point is from 24.2 to 28.3 mm. The diameter of the connector at its narrowest point 36 is from 22.45 to 20.7 mm. The depth of the connector measured from its top face 38 to its opposite bottom face 40 (not including the skirt which depends from the bottom face) is from 19.75 to 22.25 mm. The angle between the wall of the connector and its axis (which is half of the angle defined by the diametrically opposite walls of the connector) is 5°. The connector is therefore a snug fit in the bore 26 in the head part, with the top face 38 located within the bore 26 in the head part, and the bottom face 40 located adjacent to the reverse face 24 of the head part. When the connector is fully received in the bore 26 in the head part, the length of the contacting surfaces of the connector and the bore, measured along the axis of the bore, is from 19.75 to 22.25 mm. The widest point at which the connector is in contact with the bore is at the widest part of the connector part (that is at the bottom face 40). Accordingly, the ratio of the length of the contacting surfaces of the bore in the head part and the connector part when assembled, measured along the axis of the bore in the head part, to the diameter of the bore in the head part at the widest point at which it contacts the external surface of the connector part, is 1.23 (24.2:19.75) or 1.27 (28.3:22.25) in the two embodiments which are discussed.

The connector 30 has a bore 42 within it extending from the bottom face 40. The bore is tapered inwardly in a direction away from the bottom face of the connector. The bore is open at its opposite narrow end. The bore can be blind at its narrow end. A skirt 43 surrounds the bore at its open end on the bottom face 40.

The bore 42 in the connector is sized so that the spigot 14 on the stem is a snug fit within it.

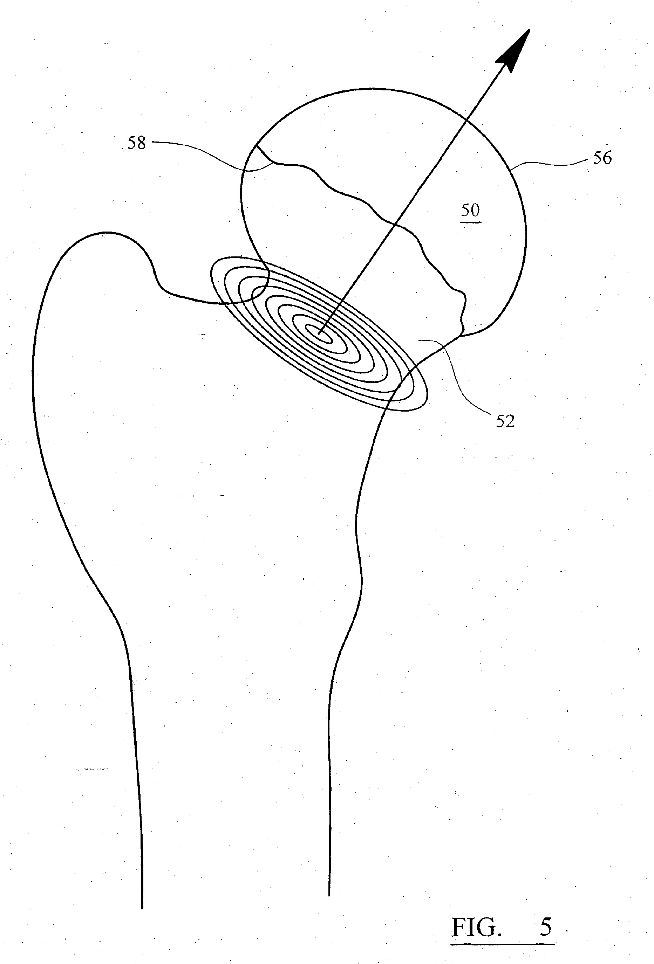

Figure 5 shows the head portion of a femur prior to any resection step in a procedure for replacement of a hip joint. The femur has a head part 50 and a neck 52 which extends between the head part and the femoral shaft 54. The outer bearing surface 56 of the head part is smooth, for articulation with a corresponding bearing surface within the acetabulum, and extends over the head part towards the femoral shaft to a boundary line 5 8. The bearing surface of the head part is defined by part of a sphere. The axis of the head part passes through the centre of the sphere, in a direction which is perpendicular to the plane which is defined by the boundary line 58.

The femoral neck 52 defines an axis which extends along its central core, between the femoral shaft and the head part.

The head part 50 of the femur can be offset relative to the femoral neck. A translational offset arises when there is a gap between the axis of the head part and the axis of the femoral neck. The size of the gap between the axes can be different from one patient to another, for example in the range 0 to 10 mm. The direction in which the axes are offset can vary, around the axis of the femoral neck.

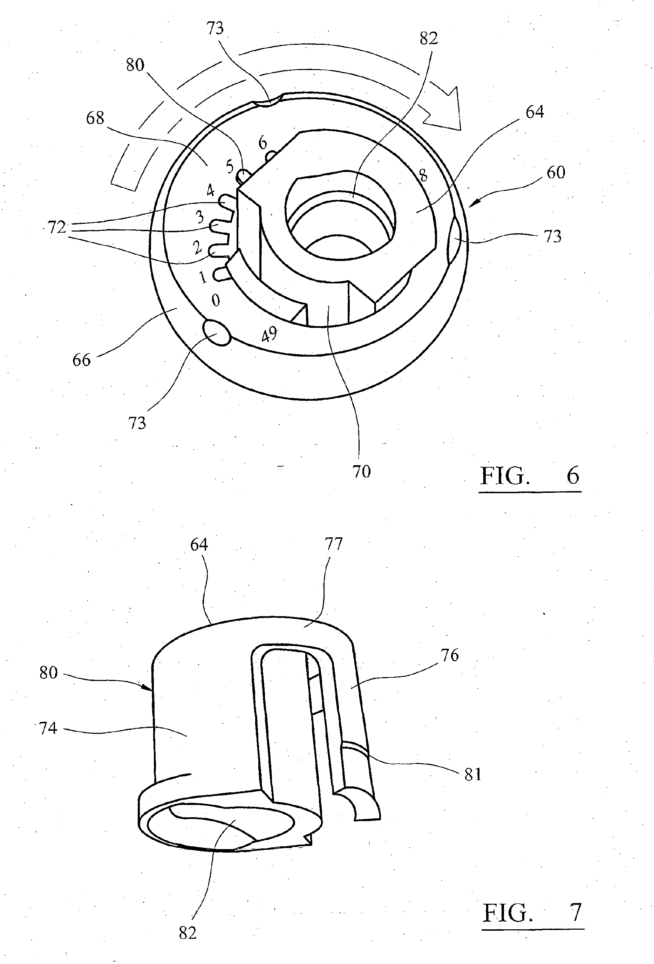

Figure 6 shows an instrument 60 which can be used to trial the head part (with its connector) on an implanted stem part. The instrument comprises a trial head part 62 and a trial connector 64. The trial connector is shown in Figure 7. The trial head part has a spherical outer surface 66 which corresponds to the bearing surface of the head part of the ultimate implant, and an opposite reverse face 68. The head part has a recess 70 within it extending inwardly from the reverse face towards the bearing surface. The recess is generally round when The recess has a plurality of grooves 72 in its side wall extending parallel to the axis of the recess. The trial head part can be formed from a metal such as a stainless steel or from a polymeric material.

The spherical outer surface 66 of the trial head part has three notches 73 at spaced apart points. The notches are distinguishable from one another, for example by means of distinguishing markings located adjacent to the notches.

The trial connector 64 is formed from a polymeric material. It comprises a body part 74 and a trigger 76 which is connected to the body part at one end 77. The material of the trigger 76, and of the body part when the trial connector is formed as a single piece) is sufficiently resilient that the trigger can be deformed inwardly towards the body part.

The body part has a rib 80 which is dimensioned so that it can fit into one of the grooves 72 in the side wall of the recess.

The trial head part and the trial connector have locking features so that the connector is retained within the recess 70 in the head part when the trigger is released, and can be removed from within the recess when the trigger is deformed towards the body part. The locking features can comprise an annular groove which extends around the recess, and a rib 81 on one or each of the body part and the trigger of the trial connector. When the rib is received in the groove, the trial connector is locked against removal from the bore in the trial head part. When the trigger 76 is squeezed towards the body part 74, the trial connector is able to move transversely within the recess in the body part so that the rib can be withdrawn from the groove, allowing the trial connector to be withdrawn from within the recess.

The body part 74 of the trial connector has a bore 82 formed in it. The bore is tapered inwardly in a direction away from the bottom face of the connector. The bore is open at its opposite narrow end. The bore is blind at its narrow end. The bore 82 in the trial connector is sized so that the spigot 14 on the stem is a snug fit within it.

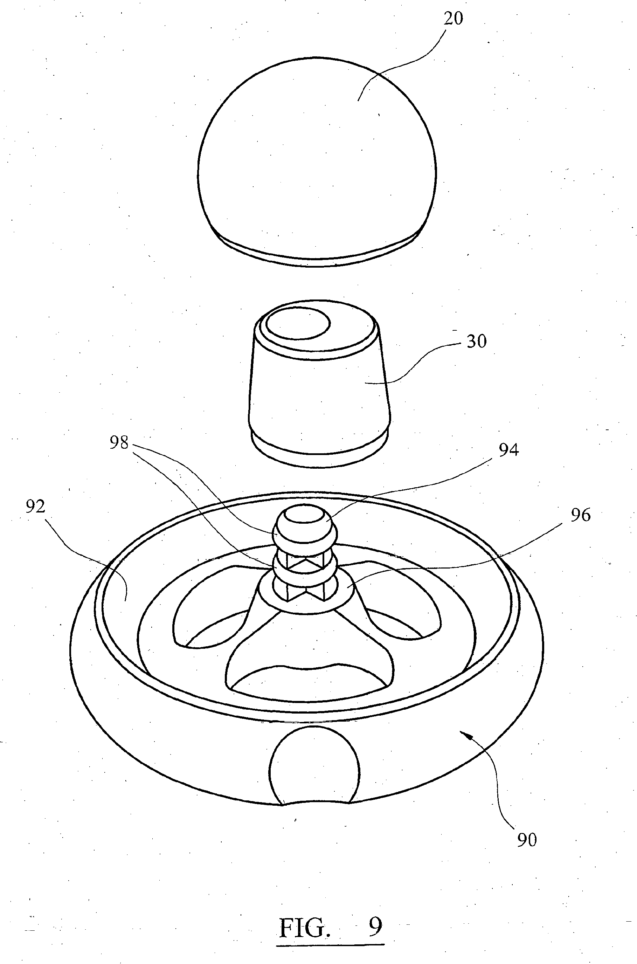

Figure 9 shows an assembly tool 90 which can be used in the assembly of the head part 20 of the femoral component and the connector 30. The tool comprises a base 92 having an upstanding spigot 94. The spigot has a collar 96 around it, which presents an upwardly facing surface. A pair of compressible O-rings 98 are provided on the spigot, located in annular grooves therein. The sizes of the spigot and the O-rings are such that the O-rings are compressed on contact with the internal wall of the bore 42 in the connector 30 when the connector is seated on the tool with the bottom face of the skirt 43 in contact with the collar 96 on the tool. This can help to retain the connector on the spigot, by virtue of the friction forces between the O-rings and the internal surface of the bore in the connector.

The assembly tool 90 is made from stainless steel. It can have a ring of a rubber material located in a groove in its lower face such that it protrudes from the groove to engage the surface on which the tool is placed when in use.

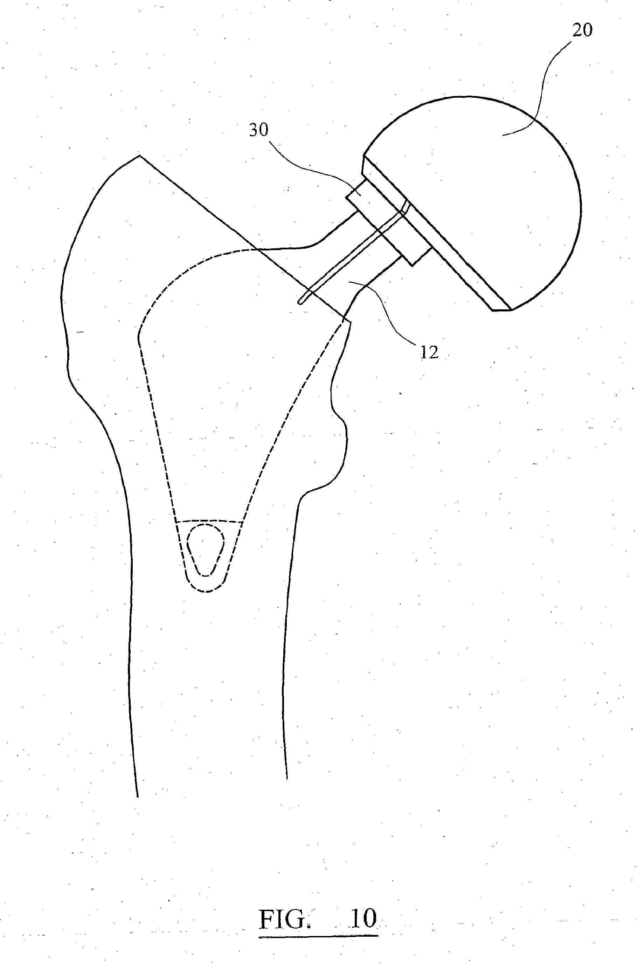

Figure 10 shows the femoral component of a hip joint prosthesis according to the present invention which has been assembled. The assembled femoral component comprises the head part 20, with the connector 30 located in the bore 26 therein. The spigot 14 on the stem part 12 of the femoral component is located in the bore 42 in the connector.

A procedure in which the invention can be implemented to provide a femoral component of a hip joint prosthesis can include the following steps.

Initial steps involve preparing the femur to receive the stem part. These steps are conventional, and include resection of the neck and head of the femur, and working on the intramedullary cavity in the femoral shaft so that it is appropriately dimensioned to receive the stem part.

Preparatory work on the patient might provide information as to the desired offset of the femoral head. The trial components described above with reference to Figures 4 to 6 can allow offsets to be assessed. Variations in the size of the gap between the axis of the head part and the axis of the femoral neck can be replicated by changing the angular relationship between the trial head part 62 and the trial connector 64, using the trigger to release the trial connector for movement in the recess in the trial head part. Variations in the direction in which the axes are offset can vary, around the axis of the femoral neck, can be replicated by rotating the trial components around the spigot 14 on the stem part 12.

Markings on the reverse face 68 of the trial head part 62 provide an indication of the size of the offset, which is then to be incorporated in the assembled head component.

A record of the angular orientation of the trial head part about the spigot 14 is made with reference to a selected one of the notches 73 on the spherical outer surface 66 of the trial head part, using a diathermy 83 to make a mark on bone tissue 84 immediately below the selected notch.

The size of the offset that is determined using the trial head part and the trial connector are reproduced in the head component with reference to the markings 28 on the reverse face 24 of the head part 20 (which are the counterparts to the grooves 72 in the side wall of the recess 70 in the reverse face 68 of the trial head part 62), and to a marking on the connector 30 (which is the counterpart to the rib 80 on the trial connector 64). The head part 20 and the connector 40 of the implant are assembled accordingly, and placed on the spigot 94 of the assembly tool 90. An impaction force is applied to the head part through an appropriate protector (such as a block of polyethylene which is configured to be a conforming fit on the bearing surface 22 of the head part 20). Application of the impaction force causes the connector to be forced downwardly on to the spigot 94 until the skirt 43 on the bottom face of the connector contacts the collar 96 on the tool, compressing the O-rings 98 on the spigot as necessary. When the skirt on the connector contacts the collar on the tool in this way, applied impaction force leads to securing of the connection between the head part 20 and the connector 40.

The assembled head component (comprising the head part 20 and the connector 40) is positioned on the spigot 14 on the stem part 12. The alignment of the head component on the stem part offset that is determined using the trial head part and the trial connector are reproduced in the head component with reference to a selected one of the markings 29 on the chamfer surface 28 which corresponds to the selected notch on the trial head part which was used previously to make a mark on the bone using the diathermy.

An impaction force is applied to the head component through an appropriate protector (such as a block of polyethylene which is configured to be a conforming fit on the bearing surface 22 of the head part 20) to cause the head component to become secured to the stem part. This is in line with existing assembly techniques for use with orthopaedic joint prostheses.

## Claims

1. An assembly for use in a hip joint replacement procedure, which comprises:
a. a stem part (12) for location in the intramedullary cavity of a femur, having a tapered spigot (14) at one end,
b. a head part (20) having a bearing surface (22) defined by a part of a sphere which subtends an angle at the centre of the sphere of at least about 180°, and a tapered bore (26) formed in it in which the spigot is a snug fit so that the tapered surfaces of the spigot and the bore are in surface to surface contact,
in which, when the spigot is received in the bore in the head part, the distance from (i) the point on the tapered surface of the spigot at or towards its free end at which the tapered surface thereof no longer contacts the tapered surface of the bore, to (ii) the centre of the sphere which is defined by the bearing surface measured from the said point on the spigot parallel to the axis of the spigot towards the intramedullary cavity, is at least about 5 mm, with the said point on the tapered surface of the spigot being located between the centre of the sphere and the bearing surface of the head part.

2. An assembly as claimed in claim 1, in which, when the spigot is received in the bore in the head part, the ratio of (i) the distance from the point on the spigot at or towards its free end where the tapered surface thereof contacts the tapered surface of the bore to the centre of the sphere which is defined by the bearing surface, measured from the said point on the spigot parallel to the axis of the spigot towards the intramedullary cavity, to (ii) the radius of the sphere which is defined by the bearing surface, is at least about 0.3.

3. An assembly for use in a hip joint replacement procedure, which comprises:
a. a stem part for location in the intramedullary cavity of a femur, having a tapered spigot at one end,
b. a head part having a bearing surface defined by a part of a sphere which subtends an angle at the centre of the sphere of at least about 180°, and a tapered bore formed in it in which the spigot can be received,
in which, when the spigot is received in the bore in the head part, the ratio of (i) the distance from the point on the spigot at or towards its free end where the tapered surface thereof contacts the tapered surface of the bore to the centre of the sphere which is defined by the bearing surface, measured from the said point on the spigot parallel to the axis of the spigot towards the intramedullary cavity, to (ii) the radius of the sphere which is defined by the bearing surface, is at least about 0.3.

4. An assembly as claimed in claim 3, in which, when the spigot is received in the bore in the head part, the distance from (i) the point on the tapered surface of the spigot at or towards its free end at which the tapered surface thereof no longer contacts the tapered surface of the bore, to (ii) the centre of the sphere which is defined by the bearing surface measured from the said point on the spigot parallel to the axis of the spigot towards the intramedullary cavity, is at least about 5 mm,

5. An assembly as claimed in claim 1 or claim 3, in which the radius of the sphere which is defined by the bearing surface is at least about 7 mm.

6. An assembly as claimed in claim 1 or claim 3, in which the radius of the sphere which is defined by the bearing surface is not more than about 20 mm.

## Patentansprüche

1. Anordnung zur Verwendung in einem Hüftgelenkaustauschverfahren, die umfasst:
a. ein Schaftteil (12) zur Anordnung in dem Markraum eines Femurs, das einen konischen Zapfen (14) an einem Ende aufweist,
b. ein Kopfteil (20) mit einer Lagerfläche (22), die von einem Teil einer Kugel definiert ist, die einen Winkel im Zentrum der Kugel von mindestens circa 180° aufspannt, und einer darin ausgebildeten konischen Bohrung (26), in der sich der Zapfen in Passsitz befindet, so dass die konischen Flächen des Zapfens und der Bohrung in Fläche-zu-Fläche-Kontakt stehen,
wobei, wenn der Zapfen der Bohrung in dem Kopfteil aufgenommen ist, die Distanz von (i) dem Punkt auf der konischen Fläche des Zapfens an oder in Richtung zu seinem freien Ende, an dem die konische Fläche desselben die konische Fläche der Bohrung nicht länger kontaktiert, zu (ii) dem Zentrum der Kugel, die von der Lagerfläche definiert ist, gemessen von dem Punkt auf dem Zapfen parallel zur Achse des Zapfens in Richtung zum Markraum, mindestens circa 5 mm beträgt, wobei sich der Punkt auf der konischen Fläche des Zapfens zwischen dem Zentrum der Kugel und der Lagerfläche des Kopfteils befindet.

2. Anordnung nach Anspruch 1, wobei, wenn der Zapfen in der Bohrung in dem Kopfteil aufgenommen ist, das Verhältnis von (i) der Distanz von dem Punkt auf dem Zapfen an oder in Richtung zu seinem freien Ende, wo die konische Fläche desselben die konische Fläche der Bohrung kontaktiert, zum Zentrum der Kugel, die von der Lagerfläche definiert ist, gemessen von dem Punkt auf dem Zapfen parallel zur Achse des Zapfens in Richtung zum Markraum, zu (ii) dem Radius der Kugel, die von der Lagerfläche definiert ist, mindestens circa 0,3 beträgt.

3. Anordnung zur Verwendung in einem Hüftgelenkaustauschverfahren, die umfasst:
a. ein Schaftteil zur Anordnung in dem Markraum eines Femurs, das einen konischen Zapfen an einem Ende aufweist,
b. ein Kopfteil mit einer Lagerfläche, die von einem Teil einer Kugel definiert ist, die einen Winkel im Zentrum der Kugel von mindestens circa 180° aufspannt, und einer darin ausgebildeten konischen Bohrung, in der der Zapfen aufgenommen werden kann,
wobei, wenn der Zapfen in der Bohrung in dem Kopfteil aufgenommen ist, das Verhältnis von (i) der Distanz von dem Punkt auf dem Zapfen an oder in Richtung zu seinem freien Ende, wo die konische Fläche desselben die konische Fläche der Bohrung kontaktiert, zum Zentrum der Kugel, die von der Lagerfläche definiert ist, gemessen von dem Punkt auf dem Zapfen parallel zur Achse des Zapfens in Richtung zum Markraum, zu (ii) dem Radius der Kugel, die von der Lagerfläche definiert ist, mindestens circa 0,3 beträgt.

4. Anordnung nach Anspruch 3, wobei, wenn der Zapfen in der Bohrung in dem Kopfteil aufgenommen ist, die Distanz von (i) dem Punkt auf der konischen Fläche des Zapfens an oder in Richtung zu seinem freien Ende, an dem die konische Fläche desselben die konische Fläche der Bohrung nicht länger kontaktiert, zu (ii) dem Zentrum der Kugel, die von der Lagerfläche definiert ist, gemessen von dem Punkt auf dem Zapfen parallel zur Achse des Zapfens in Richtung zum Markraum, mindestens circa 5 mm beträgt.

5. Anordnung nach Anspruch 1 oder Anspruch 3, wobei der Radius der Kugel, die von der Lagerfläche definiert ist, mindestens circa 7 mm beträgt.

6. Anordnung nach Anspruch 1 oder 3, wobei der Radius der Kugel, die von der Lagerfläche definiert ist, nicht mehr als circa 20 mm beträgt.

## Revendications

1. Ensemble pour utilisation dans une procédure de remplacement de l'articulation de la hanche, qui comprend :
a. une partie de tige (12) destinée à être placée dans la cavité intramédullaire d'un fémur, ayant un manchon conique (14) à une extrémité,
b. une partie de tête (20) ayant une surface de support (22) définie par une partie d'une sphère qui sous-tend un angle au centre de la sphère d'au moins environ 180°, et un alésage conique (26) ménagé dans celle-ci dans laquelle le manchon est ajusté d'une manière serrée de sorte que les surfaces coniques du manchon et de l'alésage soient en contact de surface à surface,
dans lequel, lorsque le manchon est reçu dans l'alésage dans la partie de tête, la distance (i) du point sur la surface conique du manchon à ou vers son extrémité libre où la surface conique ne reste plus en contact avec la surface conique de l'alésage, (ii) au centre de la sphère qui est définie par la surface de support mesurée depuis ledit point sur le manchon parallèle à l'axe du manchon vers la cavité intramédullaire, est au moins d'environ 5 mm, ledit point sur la surface conique du manchon se situant entre le centre de la sphère et la surface de support de la partie de tête.

2. Ensemble selon la revendication 1, dans lequel, lorsque le manchon est reçu dans l'alésage dans la partie de tête, le rapport de (i) la distance du point sur le manchon à ou vers son extrémité libre où sa surface conique vient en contact avec la surface conique de l'alésage au centre de la sphère qui est définie par la surface de support, mesurée depuis ledit point sur le manchon parallèle à l'axe du manchon vers la cavité intramédullaire, à (ii) le rayon de la sphère qui est définie par la surface de support, est au moins d'environ 0,3.

3. Ensemble pour utilisation dans une procédure de remplacement de l'articulation de la hanche, qui comprend :
a. une partie de tige destinée à être placée dans la cavité intramédullaire d'un fémur, ayant un manchon conique à une extrémité,
b. une partie de tête ayant une surface de support définie par une partie d'une sphère qui soustend un angle au centre de la sphère d'au moins environ 180°, et un alésage conique ménagé dans celle-ci dans lequel le manchon peut être reçu,
dans lequel, lorsque le manchon est reçu dans l'alésage dans la partie de tête, le rapport (i) de la distance du point sur le manchon à ou vers son extrémité libre où sa surface conique vient en contact avec la surface conique de l'alésage au centre de la sphère qui est définie par la surface de support, mesurée depuis ledit point sur le manchon parallèle à l'axe du manchon vers la cavité intramédullaire, (ii) au rayon de la sphère qui est définie par la surface de support, est au moins d'environ 0,3.

4. Ensemble selon la revendication 3, dans lequel, lorsque le manchon est reçu dans l'alésage dans la partie de tête, la distance (i) du point sur la surface conique du manchon à ou vers son extrémité libre à laquelle sa surface conique n'est plus en contact avec la surface conique de l'alésage, (ii) au centre de la sphère qui est définie par la surface de support mesurée depuis ledit point sur le manchon parallèle à l'axe du manchon vers la cavité intramédullaire, est au moins d'environ 5 mm.

5. Ensemble selon la revendication 1 ou la revendication 3, dans lequel le rayon de la sphère qui est définie par la surface de support est au moins d'environ 7 mm.

6. Ensemble selon la revendication 1 ou la revendication 3, dans lequel le rayon de la sphère qui est définie par la surface de support n'est pas supérieur à environ 20 mm.
